# EUROPEAN PATENT APPLICATION

(11) **EP 4 219 797 A1**
(43) Date of publication of application: **02.08.2023**
(21) Application number: 21872584.4
(22) Date of filing: 24.09.2021
(51) Int. Cl.: C25D 5/02, C25D 5/18, H01M 4/86, H01M 4/88, H01M 4/90, H01M 4/92, H01M 4/96, H01M 8/16, B32B 7/025, C12N 9/02, C12N 9/06, C12N 11/14, B32B 3/14, C25B 11/073, G01N 27/327

(54) **STRUCTURE, METHOD FOR PRODUCING STRUCTURE, AND ELECTROCHEMICAL DEVICE**

(30) Priority: 24.09.2020 JP 2020160050
(71) Applicant: University of Tsukuba, Ibaraki 305-8577 (JP); Université Grenoble Alpes, 38400 Saint-Martin-d'Hères (FR); Centre National de la Recherche Scientifique, 75016 Paris Cedex 16 (FR); Institut Polytechnique de Grenoble, 38000 Grenoble (FR)
(72) Inventor: TSUJIMURA, Seiya, Tsukuba-shi, Ibaraki 503-8577 (JP); NAKAGAWA, Yuto, Tsukuba-shi, Ibaraki 503-8577 (JP); ABDELKADER, Zebda, 38400 Saint-Martin-d'Heres (FR); ZELSMANN, Marc, 38400 Saint-Martin-d'Heres (FR)
(74) Representative: Hautier IP
(86) International application number: PCT/JP2021/035190
(87) International publication number: WO 2022/065455

(57) **Abstract**

A structure, comprising an electroconductive substrate and a metal deposit that is disposed on the electroconductive substrate in an island-like manner.

## Description

### [Technical Field]

The present invention relates to a structure, a method for producing a structure, and an electrochemical device.

### [Background Art]

Research and development has been conducted with respect to electrochemical devices including biofuel cells, which generate power using enzymes as a catalyst for an electrode and biomass resources such as sugars or alcohols as a fuel.

Generally, electrochemical devices utilizing a catalytic action of enzymes has an anode that includes an enzyme that promotes oxidation of a fuel, and a cathode that includes an enzyme that promotes reduction of oxygen. Electrons that have been collected by oxidation of a fuel at an anode (for example, a change from glucose to gluconolactone) are transferred to a cathode, and used to reduce oxygen to produce water (H₂O). The migration of electrons during this process is utilized for power generation or the like.

Electrochemical devices utilizing a catalytic action of enzymes have features such as being simple in configuration, operable at room temperature, environmentally and biologically compatible, and being configured from materials that are easy to obtain. Therefore, the use of electrochemical devices as biosensors and the like has been considered, in addition to as power sources (see, for example, Patent Document 1).

[Patent Document 1] International Publication No. 2018/062419

### [Summary of Invention]

### [Problem to be Solved by Invention]

In electrochemical devices utilizing a catalytic action of enzymes, improvement in the ability to reduce oxygen is an important issue in order to increase the power output.

In view of the foregoing, the present invention aims to provide a structure that exhibits an excellent ability to reduce oxygen; a method for producing a structure that exhibits an excellent ability to reduce oxygen; and an electrochemical device using the structure.

### [Means for Implementing Invention]

The means for implementing the present invention includes the following embodiments.
<1> A structure, comprising an electroconductive substrate and a metal deposit that is disposed on the electroconductive substrate in an island-like manner.
<2> The structure according to <1>, wherein the metal deposit has an average diameter of from 5 nm to 100 nm.
<3> The structure according to <1> or <2>, wherein the metal deposit comprises at least one selected from the group consisting of gold, copper, platinum and palladium.
<4> The structure according to any one of <1> to <3>, wherein the electroconductive substrate comprises a carbon material.
<5> The structure according to any one of <1> to <4>, wherein the structure further comprises an enzyme that promotes reduction of oxygen.
<6> The structure according to <5>, wherein the enzyme comprises at least one selected from the group consisting of bilirubin oxidase, laccase and polyphenol oxidase.
<7> The structure according to any one of <1> to <6>, which is used as an electrode of an electrochemical device.
<8> The structure according to <7>, wherein the electrochemical device is a direct electron transfer-type device.
<9> A method of producing the structure according to any one of <1> to <8>, the method comprising depositing a metal on an electroconductive substrate, wherein the depositing is performed by reducing metal ions under a condition in which electrolysis of water occurs.
<10> The method of producing the structure according to <9>, wherein the reducting is performed by chrono amperometry.
<11> The method of producing the structure according to <9>, wherein the reducting is performed by pulse potentiometry.
<12> The method of producing the structure according to <9>, wherein the reducting is performed by chrono amperometry and pulse potentiometry.
<13>. An electrochemical device, comprising the structure according to any one of <1> to <8>.
<14> The electrochemical device according to <13>, which is a biofuel cell, a bioreactor or a biosensor.

### [Effect of Invention]

According to the present invention, a structure that exhibits an excellent ability to reduce oxygen; a method for producing a structure that exhibits an excellent ability to reduce oxygen; and an electrochemical device using the structure are provided.

### [Brief Explanation of Drawings]

Fig. 1 is an FE-SEM image of the structure prepared in the Examples.
Fig. 2 is a result of electrochemical measurement of the structures prepared in the Examples.
Fig. 3 is an FE-SEM image of the structure prepared in the Examples.
Fig. 4 is a result of electrochemical measurement of the structures prepared in the Examples.
Fig. 5 is an FE-SEM image of the structure prepared in the Examples.
Fig. 6 is a result of electrochemical measurement of the structures prepared in the Examples.

### [Embodiments for Implementing Invention]

Embodiments for carrying out the present invention will now be described in detail. However, the invention is not limited to the following embodiments.

In the following embodiments, constituent elements (including element steps and the like) of the embodiments are not essential, unless otherwise specified. Likewise, numerical values and ranges thereof are not intended to restrict the invention.

In the present disclosure, any numerical range described using the expression "from * to *" represents a range in which numerical values described before and after the "to" are included in the range as a minimum value and a maximum value, respectively.

### <Structure>

The structure of the present disclosure includes an electroconductive substrate and a metal deposit that is disposed on the electroconductive substrate in an island-like manner.

Research conducted by the present inventors has proved that an ability of an enzyme to reduce oxygen is improved by fixing the enzyme to a structure having a configuration in which a metal deposit is disposed on an electroconductive substrate in an island-like manner, as compared with a case in which a metal deposit is not disposed on an electroconductive substrate in an island-like manner. While having not yet been clarified, the reason for this is thought to be as follows.

The efficiency of an enzyme to reduce oxygen may be improved by, in addition to increasing the amount of an enzyme to be fixed to a structure, facilitating the action of an enzyme to reduce oxygen. For example, shortening of a distance between an active center in a molecule of the enzyme and a surface of an electroconductive substrate facilitates the supply of electrons from the electroconductive substrate to the active center. As a result, it is thought that an enzyme readily reduces oxygen, thereby improving the ability of the enzyme to reduce oxygen.

In the structure of the present disclosure, a metal deposit is disposed on an electroconductive substrate in an island-like manner. Therefore, an enzyme is fixed to an electroconductive substrate such that the enzyme is disposed at a gap formed among the metal deposits. In that case, it is thought that the enzyme is fixed with its active center being located adjacent to a surface of the electroconductive substrate due to an electrostatic interaction created between the active center and a surface of an electroconductive substrate, and an electrostatic interaction created between the active center and a metal deposit.

For example, bilirubin oxidase, which is an enzyme that promotes reduction of oxygen, has a copper ion as an active center at a position adjacent to a surface of a molecule of the enzyme. Further, while bilirubin oxidase has a region around the active center being positively charged, its molecule is negatively charged as a whole. On the other hand, a surface of an electroconductive substrate is negative charged, while a surface of a metal deposit is positively charged. As a result, bilirubin oxidase is fixed at a surface of an electroconductive substrate such that the active center thereof is located adjacent to a surface of the electroconductive substrate, i.e., bilirubin oxidase readily promotes reduction of oxygen, due to an electrostatic interaction created between the active center and a surface of an electroconductive substrate and an electrostatic interaction created between a region other than the active center and a metal deposit.

Another possible reason for the improved ability of an enzyme to reduce oxygen may be that a metal deposit has fine projections, and the projections promote the reduction of oxygen by mediating the supply of electrons to an active center that is located away from a surface of an electroconductive substrate.

As described above, it is thought that the structure of the present disclosure exhibits an excellent ability to reduce oxygen as a result of regulating a molecular direction (orientation) of an enzyme to be fixed to an electroconductive substrate, and shortening a distance between an active center of an enzyme and an electroconductive substrate.

Accordingly, the structure of the present disclosure may be suitably used as an electrode of a direct electron transfer-type (DET) electrochemical device, in which electrons are directly supplied from an electroconductive substrate to an enzyme without the help of a mediator.

In the following, configuration of the structure of the present disclosure is explained.

### (Electroconductive substrate)

The material for the electroconductive substrate is not particularly limited, and may be selected from any electroconductive materials such as carbon materials and metals.

From the viewpoint of regulating the orientation of an enzyme to be fixed to a surface of the electroconductive substrate, at least a surface of the electroconductive substrate is preferably formed from a carbon material.

Examples of the carbon material include graphite, carbon black (such as ketjen black and acetylene black), MgO-template carbon particles, and carbon nanomaterials. The electroconductive substrate may include a single kind of carbon material or two or more kinds in combination.

From the viewpoint of increasing the amount of an enzyme to be fixed to a surface of an electroconductive substrate, the electroconductive substrate preferably includes a carbon material having a particulate form (carbon particles), more preferably carbon particles having pores (porous carbon particles).

From the viewpoint of readily fixing an enzyme inside a pore, porous carbon particles preferably has an average pore size of 10 nm or more, more preferably 50 nm or more, further preferably 100 nm or more.

From the viewpoint of securing a sufficient degree of specific surface area, the average pore size is preferably 1000 nm or less, more preferably 500 nm or less, further preferably 300 nm or less.

The average pore size refers to an arithmetic average value of maximum diameters of arbitrarily selected 100 pores for measurement.

The particle size of the porous carbon particles may be determined depending on the type of an enzyme to be fixed, the type of an electroconductive substrate, and the like. For example, the particle size of the porous carbon particles may be from 0.1 µm to 5 µm or from 0.5 µm to 1 µm.

The particle size refers to a particle size measured at 50% accumulation from the small-size side in a volume-based particle size distribution obtained by a laser diffraction/scattering method (D50).

Specific examples of the porous carbon particles include MgO-template carbon particles. The MgO-template carbon particles are porous carbon particles produced by sintering a mixture of a carbon precursor and magnesium oxide (MgO) or MgO precursor, and removing MgO particles from a sintered product of the mixture. According to this process, pores with a uniform size and a uniform shape are formed at portions from which MgO particles have been removed, whereby carbon particles in which uniform pores are regularly disposed are obtained.

The carbon precursor used in the production of MgO-template carbon particles is not particularly limited, as long as it contains carbon atoms and has an ability to carbonize when heated.

Examples of the carbon precursor include resins, pitches, condensed polycyclic hydrocarbon compounds such as naphthalene, phenanthrene and anthracene, condensed heterocyclic compounds such as indole, isoindole and quinoline, and derivatives of these compounds.

The MgO precursor used in the production of MgO-template carbon particles is not particularly limited, as long as it produces MgO particles by thermal decomposition when heated. Examples of the MgO precursor include magnesium hydroxide, magnesium carbonate, basic magnesium carbonate, and organic acid salts of magnesium such as magnesium acetate, magnesium citrate, magnesium oxalate and magnesium gluconate.

When an electroconductive substrate includes porous carbon particles, the porous carbon particles may form a surface layer of the electroconductive substrate. In that case, the material for a portion of the electroconductive substrate other than a surface layer is not particularly limited, and may be selected from any electroconductive materials such as carbon particles and metals, for example, a carbon paper having gas diffusivity.

### (Metal deposit)

The material for the metal deposit to be disposed on an electroconductive substrate in an island-like manner is not particularly limited. From the viewpoint of avoiding the elusion or the like during use, the material is preferably selected from metals having a smaller ionization tendency such as gold, copper, platinum and palladium. The metal deposit may be formed from a single kind of metal or two or more kinds in combination.

In the present disclosure, "being disposed on an electroconductive substrate in an island-like manner" refers to a state in which, at a surface of an electroconductive substrate, a region at which a metal deposit is present (island) and a region at which a metal deposit is not present are intermixed, rather than a state in which a metal deposit is present at an entire surface of the electroconductive substrate.

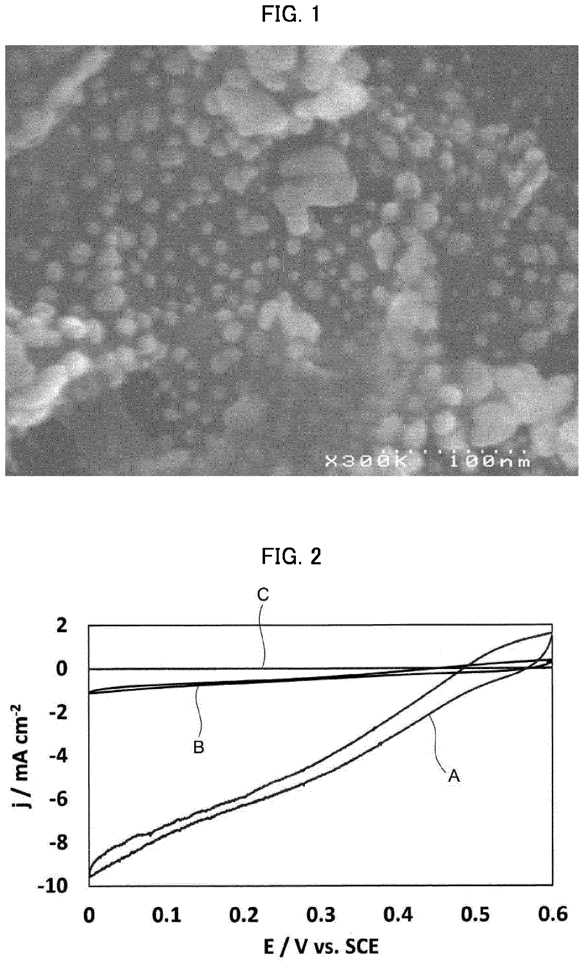

An exemplary state of a surface of an electroconductive substrate on which a metal deposit is disposed in an island-like manner is shown in Fig. 1, a field emission-scanning electron microscope (FE-SEM) image of a surface of a structure prepared in the Examples. In Fig. 1, a relatively bright region refers to a metal deposit and a relatively dark region refers to a surface of an electroconductive substrate.

From the viewpoint of regulating the molecular orientation of an enzyme and securing the amount of an enzyme to be fixed to an electroconductive substrate, the average diameter of the metal deposit at a front view of an electroconductive substrate is preferably from 5 nm to 100 nm, more preferably 10 nm to 50 nm.

The average diameter of the metal deposit refers to an arithmetic value of maximum diameters of arbitrarily selected 100 metal deposits for measurement.

The distance between the metal deposits being adjacent to each other on an electroconductive substrate may be adjusted depending on the molecular size of an enzyme to be fixed or the like. For example, the average distance between the metal deposits being adjacent to each other is preferably from 5 nm to 100 nm, more preferably from 10 nm to 50 nm.

The average distance between the adjacent metal deposits refers to an arithmetic value of the distances between arbitrarily selected 100 pairs of metal deposits for measurement.

### (Enzyme that promotes reduction of oxygen)

The structure may include an enzyme that promotes reduction of oxygen. The type of the enzyme that promotes reduction of oxygen is not particularly limited, and examples thereof include bilirubin oxidase, laccase, polyphenol oxidase and ascorbate oxidase. Among these, bilirubin oxidase, laccase and polyphenol oxidase are preferred, and bilirubin oxidase is more preferred. The structure may include a single kind of enzyme or two or more kinds in combination.

Bilirubin oxidase is a multicopper oxidase having a copper ion at an active center. In a living organism, bilirubin oxidase catalyzes the following reaction.

2 bilirubin + O₂ → 2 biliverdin + 2 H₂O

As such, bilirubin oxidase catalyzes a reaction of reducing oxygen using an electron obtained from bilirubin, and producing water. In addition, since bilirubin oxidase has an ability to directly receive an electron from a substance other than bilirubin and use the electron to reduce oxygen, the enzyme is highly useful as a catalyst for electrochemical devices. Further, the use of bilirubin oxidase as an enzyme to be fixed to an electroconductive substrate has an advantage in terms of improving the catalytic action of an enzyme by regulating the molecular orientation thereof.

### (Organic compound)

The structure may include an organic compound having a thiol group, an amino group or a carboxy group to be disposed at a surface of a metal deposit.

When an organic compound is disposed at a surface of a metal deposit, an electric current that occurs due to reduction of oxygen tends to increase. The reason for this is thought to be that a degree of orientation of an enzyme is improved in the presence of an organic compound, which is simulating a substrate of the enzyme, wherein an electron transfer rate between an enzyme and an electrode is increased.

Among the organic compounds having a thiol group, an amino group or a carboxy group, an organic compound having a thiol group is preferred. Specific examples of the organic compound having a thiol group include mercaptobenzoic acid, mercaptopropionic acid, mercaptopropanol and propanethiol. Among these, mercaptobenzoic acid is preferred.

### (Other materials)

As necessary, the structure may include a material other than an electroconductive substrate, a metal deposit, and an enzyme.

For example, the structure may include a binder for binding a carbon material, a support for supporting an electroconductive substrate, and a mediator for promoting the transfer of electrons from an electroconductive substrate to an enzyme.

### <Method for producing the structure>

The method for producing a structure of the present disclosure includes depositing a metal on an electroconductive substrate, wherein the depositing is performed by reducing metal ions under a condition in which electrolysis of water occurs.

According to the present method, a structure including an electroconductive substrate and a metal deposit that is disposed on the electroconductive substrate in an island-like manner is produced. While having not yet been clarified, the reason for this is thought to be as follows

In the present method, reduction of metal ions is conducted under a condition at which electrolysis of water occurs. Therefore, when a reduced metal is deposited on a surface of an electroconductive substrate, air bubbles of hydrogen are generated at a surface of an electroconductive substrate due to the electrolysis of water. The depositing of a reduced metal is partially hindered by the air bubbles, whereby a metal deposit is formed on a surface of the electroconductive substrate in an island-like manner.

The present method may be conducted by any known process. For example, the present method may be conducted by applying a voltage to an aqueous solution including metal ions, which is in contact with an electroconductive substrate, under a condition at which electrolysis of water and reduction of metal ions occur.

The conditions for the application of a voltage to an aqueous solution including metal ions are not particularly limited as long as electrolysis of water and reduction of metal ions occur. For example, the application of a voltage may be conducted under the following conditions.
Metal ion concentration: from 0.1 mM to saturation
pH: from 0 to 14
Voltage: from -5V to -1V
Water temperature: from 5°C to 60°C

The reducing of metal ions may be conducted by any one of chrono potentiometry, pulse potentiometry or chrono amperometry. The reducing of metal ions may be conducted by a combination of these methods. For example, the reducing of metal ions may be conducted by a combination of chrono amperometry and pulse potentiometry. In that case, the chrono amperometry and the pulse potentiometry may be conducted in this order.

The conditions for applying a pulse current in pulse/chrono potentiometry are not particularly limited, and may be selected from 1 second to 30 minutes and 0.5 mA/cm² to 1 A/cm², for example.

The type and preferred embodiments of the electroconductive substrate and the metal used in the present method are the same as the type and preferred embodiments of the electroconductive substrate and the metal used in the structure as described above.

### <Electrochemical device>

The electrochemical device of the present disclosure include the structure as described above. The type, purpose and the like of the electrochemical device are not particularly limited, as long as the electrochemical device utilizes a catalytic action of an enzyme fixed to the structure.

Specific examples of the electrochemical device include biofuel cells, bioreactors and biosensors.

The electrochemical device may include the structure as described above as a cathode together with an anode, or the electrochemical device may include the structure alone.

In the following, a configuration of a biofuel cell as an exemplary configuration of the electrochemical device is explained.

The biofuel cell includes a fuel, the structure as described as a cathode, and an anode that includes an enzyme that promotes oxidation of a fuel.

Electrons are generated by the oxidation of the fuel at the anode and transferred to the cathode, and the electrons are used for the reduction of oxygen at the cathode. The migration of electrons during this process is utilized for power generation.

The fuel used in a biofuel cell is not particularly limited, as long as oxidation thereof is promoted by an enzyme included in an anode. Specific examples of the fuel include sugars, alcohols, aldehydes, amino acids, amines, lactic acid and uric acid.

The biofuel cell may include a single kind of fuel or two or more kinds in combination. The fuel may be a substance that is directly oxidized by an enzyme in the anode, or may be a substance that becomes oxidizable through hydrolysis or the like (for example, starch that turns to glucose by hydrolysis).

The type of the enzyme included in the anode is not particularly limited, and may be selected depending on the type of a fuel to be oxidized.

The anode may include a single kind of enzyme or two or more kinds thereof. The anode may include a combination of an enzyme that makes a fuel oxidizable by hydrolysis or the like, and an enzyme that promotes oxidation of the fuel.

Examples of the combination of an enzyme and a sugar as a fuel include glucosedehydrogenase with glucose as a fuel; fructose dehydrogenase with fructose as a fuel; a combination of invertase and glucosedehydrogenase with sucrose as a fuel; and a combination of amylase and glucosedehydrogenase with starch as a fuel.

The electroconductive substrate to which an enzyme included in an anode is fixed and other materials that may be used as necessary for the electrochemical device are not particularly limited, and may be selected from the electroconductive substrate and materials that may be used for the structure as described above. Specifically, the electrochemical device may include a binder for binding a carbon material, a support for supporting an electroconductive substrate, a mediator for promoting the transfer of electrons from an electroconductive substrate to an enzyme, and the like.

### [Examples]

In the following, the present disclosure is explained in detail by referring to the Examples. It should be noted that the present disclosure is not limited to the Examples.

### (1) Preparation of electroconductive substrate

A mixture was prepared by mixing MgO-template carbon particles (average pore size: 200 nm) and polyvinylidene fluoride as a binder with 1-methyl-2-pyrrolidone. The mixture was applied onto one surface of a gas-diffusion carbon paper, and dried at 60°C for 12 hours, thereby obtaining an electroconductive substrate.

### (2) Formation of gold deposit

A voltage was applied to the electroconductive substrate at -3V (vs. SCE) while a portion of a surface of the electroconductive substrate is in contact with an HCl aqueous solution containing HAuCl₄ (HAuCl₄ concentration: 1 mM, pH: 2.0), and gold was deposited on a surface of the electroconductive substrate. During the application of a voltage, air bubbles of hydrogen were generated in the solution due to electrolysis of water. An FE-SEM image of a surface of an electroconductive substrate with a gold deposit formed thereon is shown in Fig. 1.

As shown in Fig. 1, a gold deposit with an average diameter of approximately 10 nm was formed on a surface of the electroconductive substrate (MgO carbon particles) in an island-like manner.

### (3) Fixation of enzyme

A Nafion TBAB (tetrabutylammonium bromide) solution containing BOD (bilirubin oxidase) (BOD concentration: 15 mg/mL) was dropped onto a region of the electroconductive substrate at which a gold deposit was formed, dried to fix the BOD to the electroconductive substrate, thereby obtaining Sample A for evaluation.

Sample B was obtained by fixing the BOD to an electroconductive substrate in the same manner as Sample A, except that a gold deposit was not formed on the electroconductive substrate.

An electroconductive substrate on which a gold deposit was formed but BOD was not fixed was used as Sample C.

### The Nafion TBAB solution containing BOD was prepared by the following process.

A Nafion TBAB film is formed by mixing Nafion with TBAB in order to substitute the hydrogen ions of Nafion with TBA ions (tetrabutylammonium), and then removing excess TBAB etc. with distilled water. The Nafion TBAB film was dissolved in ethanol to prepare a solution (100 mg/mL). The Nation TBAB ethanol solution is added to a BOD solution, which is prepared with a citrate buffer solution, by an amount of 25 µL/mL.

By mixing a Nafion TBAB solution to a BOD solution, a decrease in output power caused by desorption of BOD may be avoided. Further, by substituting the hydrogen ions of Nafion with TBA ions, the BOD may be protected from being affected by a local change in pH caused by hydrogen ions.

### (4) Electrochemical measurement

Electrochemical measurement was conducted by cyclic voltammetry (CV) at a measurement range of from 0 to 600 mV and a sweep rate of 2 mV/s, by immersing a platinum electrode as a counter electrode, a saturated calomel electrode as a reference electrode, and Sample A in a citrate buffer solution (100 mM, pH 5.0).

The same measurement was conducted on Sample B, which is obtained in the same manner as Sample A except for the formation of a gold deposit, and Sample C, which is obtained in the same manner as Sample A except for the fixing of an enzyme. The results are shown in Fig. 2.

As shown in Fig. 2, Sample A, in which an enzyme is fixed following the formation of a gold deposit, exhibits a greater amount of increase in the reduction current as compared with Sample B, in which an enzyme is fixed without the formation of a gold deposit, indicating an improvement in the reductive reaction of oxygen.

In the measurement on Sample C, in which an enzyme is not fixed following the formation of a metal deposit, the reductive current remained unchanged. The result indicates that a gold deposit in itself does not contribute to the reductive reaction of oxygen.

The results of the measurement suggest that an increase in the reductive current shown in Sample A is caused by the fixation of an enzyme following the formation of a gold deposit.

### (5) Formation of gold deposit

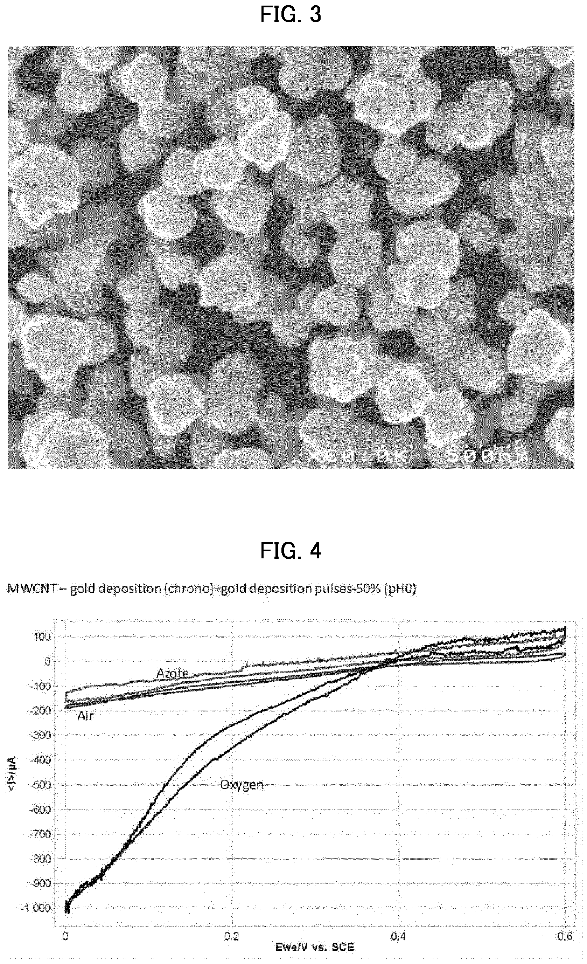

Gold was deposited on a surface of an electroconductive substrate in the same manner as (2), except that the application of a voltage was conducted by pulse potentiometry (repeat application of pulse current: 200 mA/cm² for 0.05 sec with 0A for 1 sec, total time: 10.5 min). An FE-SEM image of a surface of an electroconductive substrate at which a gold deposit is formed is shown in Fig. 3.

### (6) Fixation of enzyme

Sample D was obtained by fixing BOD in the same manner as (3) to an electroconductive substrate at which a gold deposit is formed in (5).

### (7) Electrochemical measurement

Electroconductive measurement was conducted on Sample D by cyclic voltammetry under the three conditions, namely, with nitrogen bubbling for removing oxygen (Azote), without nitrogen bubbling (Air), and with oxygen bubbling (Oxygen). The results are shown in Fig. 4.

As shown in Fig. 4, the result of the measurement with oxygen bubbling exhibits a greater amount of increase in the reductive current, indicating that the increase of the current is caused by the action of BOD to reduce oxygen.

### (8) Evaluation on surface modification of gold deposit with organic compound

An electroconductive substrate on which a gold deposit is formed in the same manner as (1) and (2) was immersed in an aqueous solution including 4-mercaptobenzoic acid or 2-mercaptobenzoic acid (10 mM), and washed with water. Thereafter, BOD was fixed to the electroconductive substrate in the same manner as (3), thereby obtaining Sample E, in which a surface of a gold deposit was modified with 4-mercaptobenzoic acid, and Sample F, in which a surface of a gold deposit was modified with 2-mercaptobenzoic acid. Sample G, in which a surface of a gold deposit was not modified with an organic compound, was prepared for comparison.

Electrochemical measurement was conducted by cyclic voltammetry (CV) at a measurement range of from 0 to 600 mV and a sweep rate of 2 mV/s, by immersing a platinum electrode as a counter electrode, a saturated calomel electrode as a reference electrode, and each of the Samples in a citrate buffer solution (100 mM, pH 5.0).

The current value at 200 mM in each of the Samples was measured, and a current index of Sample E and Sample F, with respect to a current value of Sample G given as 1, was calculated. As shown in Table 1, Sample E and Sample F, in which a surface of a gold deposit is modified with 4-mercaptobenzoic acid or 2-mercaptobenzoic acid, exhibit a greater current value as compared with Sample G without surface modification of a gold deposit.

**Table 1**

| Sample | Organic compound | Current index |
|---|---|---|
| E | 4-mercaptobenzoic acid | 2.0 |
| F | 2-mercaptobenzoic acid | 1.3 |
| G | not used | 1.0 |

### (9) Evaluation on electroconductive substrate not including carbon particles

A gas-diffusion carbon paper used in (1) was used as an electroconductive substrate without attaching MgO-template carbon particles thereto, and gold was deposited on the electroconductive substrate.

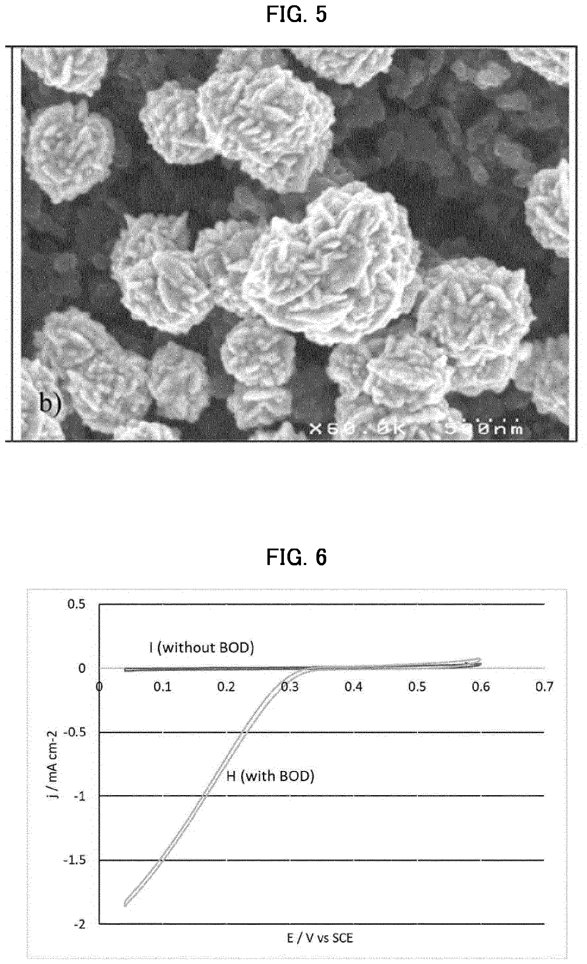

Specifically, a voltage was applied to the electroconductive substrate at -3V (vs. SCE) while a portion of a surface of the electroconductive substrate was in contact with an HCl aqueous solution containing HAuCl₄ (HAuCl₄ concentration: 2.5 mM, pH: 2.0), and the application of a voltage was stopped at 3C. Thereafter, a process of applying a current at -50 mA for 0.5 sec was conducted for 800 times with intervals of 1 sec, thereby forming a gold deposit on a surface of the electroconductive substrate. An FE-SEM image of a surface of an electroconductive substrate with a gold deposit formed thereon is shown in Fig. 5.

As shown in Fig. 5, a gold deposit was formed on a surface of the electroconductive substrate (gas-diffusion carbon paper) in an island-like manner.

After the deposit of gold, BOD was fixed to the electroconductive substrate in the same manner as (3), thereby obtaining Sample H for evaluation.

Electrochemical measurement was conducted by cyclic voltammetry (CV) at a measurement range of from 0 to 600 mV and a sweep rate of 2 mV/s, by immersing a platinum electrode as a counter electrode, a saturated calomel electrode as a reference electrode, and the Sample in a phosphate buffer solution (100 mM, pH 7.0).

The same measurement was conducted on Sample I, which was obtained in the same manner as Sample H except that BOD was not fixed to the electroconductive substrate. The results are shown in Fig. 6.

As shown in Fig. 6, a reductive current was observed at approximately 0.3V in Sample H (with BOD), whereas a reductive current was not observed in Sample I (without BOD). The results suggest that the structure of the present disclosure exhibits an excellent ability to reduce oxygen with an electroconductive substrate that does not include carbon particles.

## Claims

1. A structure, comprising an electroconductive substrate and a metal deposit that is disposed on the electroconductive substrate in an island-like manner.

2. The structure according to claim 1, wherein the metal deposit has an average diameter of from 5 nm to 100 nm.

3. The structure according to claim 1 or claim 2, wherein the metal deposit comprises at least one selected from the group consisting of gold, copper, platinum and palladium.

4. The structure according to any one of claims 1 to 3, wherein the electroconductive substrate comprises a carbon material.

5. The structure according to any one of claims 1 to 4, wherein the structure further comprises an enzyme that promotes reduction of oxygen.

6. The structure according to claim 5, wherein the enzyme comprises at least one selected from the group consisting of bilirubin oxidase, laccase and polyphenol oxidase.

7. The structure according to any one of claim 1 to claim 6, which is used as an electrode of an electrochemical device.

8. The structure according to claim 7, wherein the electrochemical device is a direct electron transfer-type device.

9. A method of producing the structure according to any one of claim 1 to claim 8, the method comprising depositing a metal on an electroconductive substrate, wherein the deposition is performed by reducing metal ions under a condition in which electrolysis of water occurs.

10. The method of producing the structure, according to claim 9, wherein the reduction is performed by chrono amperometry.

11. The method of producing the structure, according to claim 9, wherein the reduction is performed by pulse potentiometry.

12. The method of producing the structure, according to claim 9, wherein the reduction is performed by chrono amperometry and pulse potentiometry.

13. An electrochemical device, comprising the structure according to any one of claim 1 to claim 8.

14. The electrochemical device according to claim 13, which is a biofuel cell, a bioreactor or a biosensor.
